# EUROPEAN PATENT APPLICATION

(11) **EP 4 585 232 A1**
(43) Date of publication of application: **16.07.2025**
(21) Application number: 24382031.3
(22) Date of filing: 15.01.2024
(51) Int. Cl.: A61L 27/26, A61L 27/38

(54) **COMPOSITION COMPRISING MESENCHYMAL STEM CELLS AS ACTIVE SUBSTANCE EXPANDED WITHIN A CELL MATRIX**

(71) Applicant: Servicio Andaluz de Salud, 41071 Sevilla (ES); Universidad de Granada, 18071 Granada (ES)
(72) Inventor: ESPINOSA IBÁÑEZ, Olga, 18014 Granada (ES); FERNÁNDEZ GONZÁLEZ, Ana, 18014 Granada (ES); UBAGO RODRÍGUEZ, Ana Dolores, 18014 Granada (ES); QUIÑONES VICO, María Isabel, 18014 Granada (ES); LIZANA MORENO, Antonio Manuel, 18014 Granada (ES); COSTELA RUIZ, Víctor Javier, 18071 Granada (ES); FERNÁNDEZ PORCEL, Natividad, 18014 Granada (ES); GUERRERO CALVO, Jorge, 18014 Granada (ES); SIERRA SÁNCHEZ, Álvaro, 18014 Granada (ES); ARIAS SANTIAGO, Salvador, 18014 Granada (ES)
(74) Representative: Hoffmann Eitle

(57) **Abstract**

The present invention refers to a composition comprising mesenchymal stem cells as active substance expanded within a cell matrix, for use in tissue repair or tissue regeneration.

## Description

### FIELD OF THE INVENTION

The present invention refers to the medical field. Particularly, the present invention refers to a composition comprising mesenchymal stem cells as active substance expanded within a cell matrix, for use in tissue repair or tissue regeneration.

### STATE OF THE ART

Stem cells are characterized by their self-renewal ability and differentiation potential. These cells can be divided into embryonic and adult stem cells. Most adult stem cells are minor populations found in adult organs that can differentiate into specific cell types of their tissue of origin, e.g., mesenchymal stem cells. Several lines of evidence have shown that under appropriate environments, mesenchymal stem cells are able to differentiate into mesodermal, endodermal and even ectodermal cells. In addition, mesenchymal stem cells have the ability to migrate and engraft into host tissues that can help in repair and enhancement of tissue regeneration.

Mesenchymal stem cells are multipotent cells that can be found in several tissues such as bone marrow, adipose tissue, synovium, deciduous teeth, umbilical cord blood and blood vessels. Mesenchymal stem cells (MSCs) are a promising cell type for therapy, because they have the potential to differentiate into repair tissue and also have trophic and immunomodulatory capacities. Mesenchymal stem cells can also stimulate endogenous tissue repair, in addition to their ability to differentiate into cells of the mesoderm lineage. Further, growth factors secreted by MSCs, have been shown to influence tissue repair and immunological processes. Mesenchymal stem cells also possess "anti-inflammatory" properties. These cells secrete factors that mitigate inflammation and promote wound healing of normal wounds.

Although the use of mesenchymal stem cells in tissue repair or tissue regeneration has been described before, there is an unmet medical need of identifying a stable cell matrix wherein the mesenchymal stem cells can be efficiently expanded since, in the process of treating chronic wounds, it is necessary that the preparation that is deposited on the wound is stable for a minimum of 48 hours, so the product remains on the wound for the time needed to repair the tissue.

The present invention is focused on solving this problem by shortening the healing time of chronic wounds that usually requires long and time-consuming procedures. For instance, chronic wounds of the lower limb venous ulcer type present a very heterogeneous therapeutic approach in professional clinical practice. The present invention aims to provide uniformity in the treatment of this type of injury, improving the technique and reducing the time required for the tissue regeneration process of the injury. This reduces costs, improves the patient's quality of life and reduces the care burden on the staff in charge of treating this type of wound.

### DESCRIPTION OF THE INVENTION

### Brief description of the invention

The present invention refers to a composition comprising mesenchymal stem cells as active substance expanded within a cell matrix, for use in tissue repair or tissue regeneration.

The composition of the invention is characterized by a high stability (6 hours at a temperature between 0 and 8°C) so it can be used for treating chronic wounds wherein it is necessary that the preparation that is deposited on the wound is stable for a minimum of 48 hours, so the product remains stable on the wound for the time needed to repair the tissue.

So, by using the composition of the invention, it is possible to shorten the healing time of chronic wounds that usually requires long and time-consuming procedures, such as chronic wounds of the lower limb venous ulcer type which present a very heterogeneous therapeutic approach in professional clinical practice. As cited above, the present invention aims to provide uniformity in the treatment of this type of injury, improving the technique and reducing the time required for the tissue regeneration process of the injury. This reduces costs, improves the patient's quality of life and reduces the care burden on the staff in charge of treating this type of wound.

Consequently, the first embodiment of the present invention refers to a composition comprising mesenchymal stem cells as active substance expanded within a cell matrix, characterized in that the cell matrix comprises: Hyaluronic acid and fibrin.

It should be noted that the choice of the fibrin-hyaluronic acid combination for the preparation of the films is due to the fact that, in addition to allowing the growth and proliferation of human cells, it resembles the composition of native skin. Fibrin is a biopolymer involved in blood coagulation, with excellent biocompatibility, biodegradability and its interaction with some extracellular matrix (ECM) proteins leading to better cell adhesion and proliferation. Hyaluronic acid is a polysaccharide of the glycosaminoglycan type with β-bonds, which stands out for its structural function, thanks to which the film acquires the consistency of a gel. It is one of the most suitable natural materials to form part of scaffolds, due to its wide distribution in vertebrate tissues, its excellent biocompatibility and its biodegradability (it generates nontoxic degradation products). It is part of the structure of the skin that plays a fundamental role in wound healing by stimulating fibroblast proliferation, keratinocyte migration and remodelling of the ECM. In addition, when degraded, it generates a product that has a proangiogenic effect by activating signalling cascades related to CD44 receptors present in keratinocytes, facilitating their differentiation. Finally, like fibrin, it shows haemostatic capacity in wound healing.

In a preferred embodiment, the cell matrix comprises: Hyaluronic acid, human plasma (source of fibrin), tranexamic acid and CaCₗ₂; preferably also comprising culture medium and water.

The tranexamic acid is an antifibrinolytic that blocks the lysine present in the active site of plasmin, allowing the three-dimensional scaffolds generated to last longer without considerably altering their properties. Its mechanism of action lies in blocking the formation of plasmin by inhibiting the proteolytic activity of plasminogen activators, which ultimately inhibits clot dissolution (fibrinolysis). It is therefore called an antifibrinolytic (fibrinolysis inhibitor). So, its activity as an antifibrinolytic justifies its use in tissue engineering because it blocks the lysine present in the active site of plasmin allowing the generated three-dimensional scaffolds to last longer without a considerable change in their properties.

Calcium chloride is a coagulation inducer promoting the formation of the three-dimensional networks.

In a preferred embodiment the active substance comprises allogeneic adipose-derived adult mesenchymal stem cells.

In a preferred embodiment, the cell matrix comprises: Between 1 and 10 % v/v of hyaluronic acid and between 78 and 88 %v/v of human plasma.

In a preferred embodiment, the cell matrix comprises: Between 1 and 10 % v/v of hyaluronic acid, between 78 and 88 % v/v of human plasma, 2.02 % v/v of culture medium, 2.4 % v/v of tranexamic acid, 1.34 % v/v of CaCl₂ and 6.24 % v/v of water.

**Table 1**

| **EXCIPIENTES** | **COMPOSICIÓN % VOLUMEN** |
|---|---|
| **Hyaluronic acid** | 1-10% |
| **Human plasma** | 88-78% |
| **Culture medium** | 2.02% |
| **Tranexamic acid** | 2.4% |
| **2% CaCl₂** | 1.34% |
| **Water for injection** | 6.24% |

Specifically, the cell matrix comprises: 5 % v/v of hyaluronic acid, 83 % v/v of human plasma, 2.02 % v/v of culture medium, 2.4 % v/v of tranexamic acid, 1.34 % v/v of CaCl₂ and 6.24 % v/v of water.

**Table 2**

| **EXCIPIENTS** | **COMPOSITION % VOLUME (BAMSV_{TOTAL} =10 mL)** | **COMPOSITION % VOLUME** |
|---|---|---|
| Hyaluronic acid (final concentration 0.08%) | 0.5 mL | 5% |
| Human plasma | 8.3 mL | 83% |
| Culture medium | 0.624 mL | 2.02% |
| Tranexamic acid | 0.202 mL | 2.4% |
| 2% CaCl₂ | 0.240 mL | 1.34% |
| Water for injection | 0.134 mL | 6.24% |

In a preferred embodiment, the cell matrix is in the form of a gel.

In a preferred embodiment, the mesenchymal stem cells are obtained from adipose tissue, preferably from subcutaneous adipose tissue.

In a preferred embodiment, the concentration of the mesenchymal stem cells expanded within the cell matrix is between 330,000 cells/cm² and 400,000 cells/cm².

In a preferred embodiment, the concentration of the mesenchymal stem cells expanded within the matrix is 360,000 cells/cm².

In a preferred embodiment, the cell matrix is in the form of a sheet having between 2 and 144 cm² and a total amount of cells between 330,000 cells/cm² and 400,000 cells/cm².

In a preferred embodiment, the cell matrix is in the form of a sheet having 21 cm² and a total amount of 7.5×10⁶ cells.

In a preferred embodiment, the composition of the invention is for use in tissue repair or tissue regeneration.

In a preferred embodiment, the composition of the invention is characterized in that it is administered topically.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.

### Description of the figures

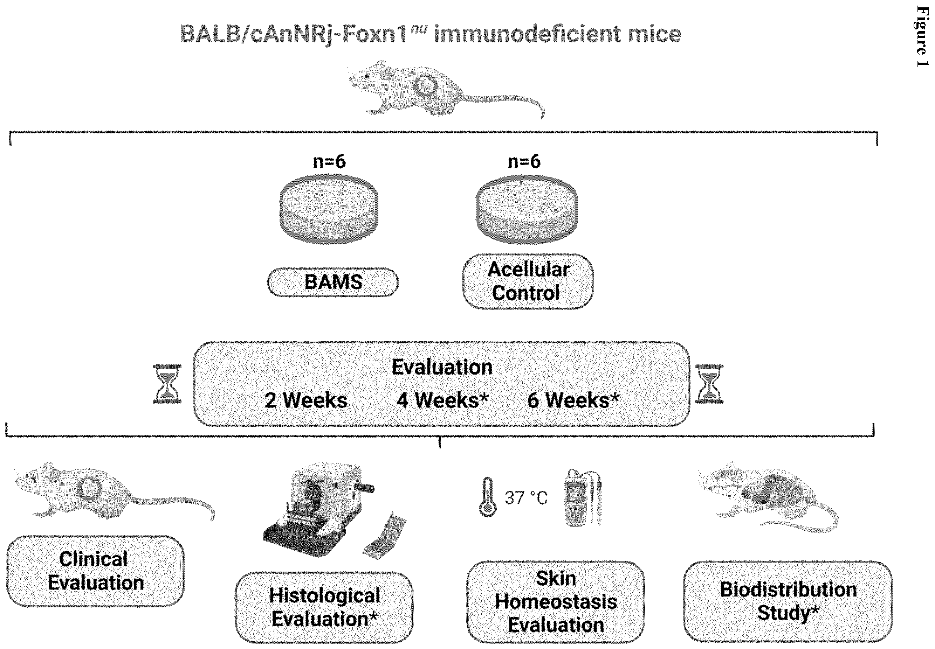

**Figure 1****.** Representative scheme of the study carried out at the preclinical level, using different treatments for an extirpation model with immunodeficient mice (BALB/cAnNRj-Foxn1*^{nu}*), comparing the results with the healthy skin of the mice themselves for 2, 4 and 6 weeks. BAMS: Bioengineered Artificial Mesenchymal Sheets.

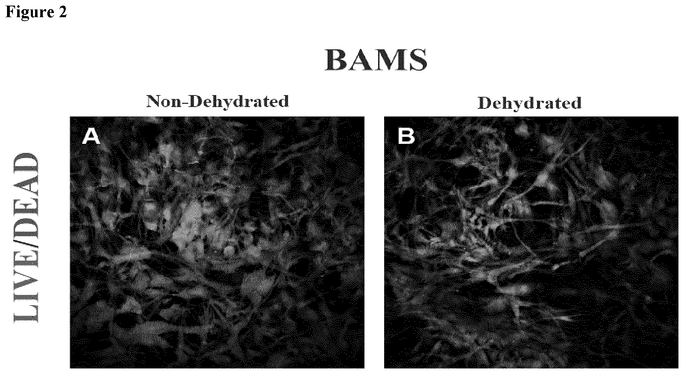

**Figure 2****.** LIVE/DEAD viability assay of non-dehydrated and dehydrated BAMS **(A)** non-dehydrated BAMS; **(B)** dehydrated BAMS. Dead cells are shown in red and live cells in green. n = 3. 10x magnification. BAMS: Bioengineered Artificial Mesenchymal Sheets.

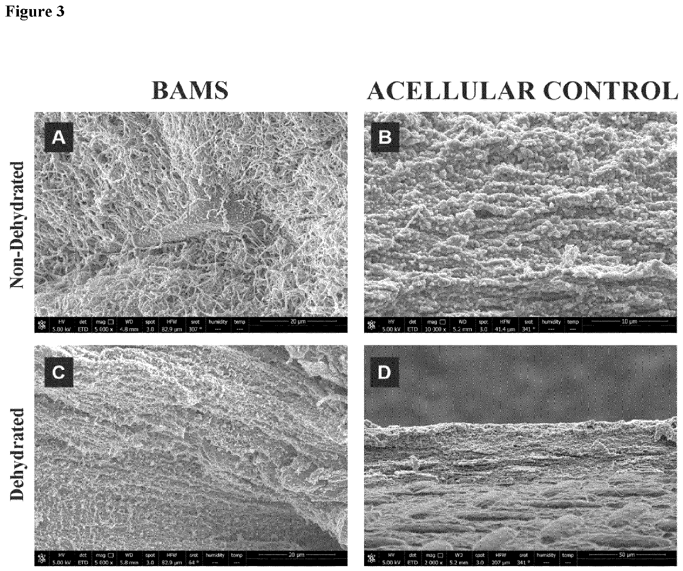

**Figure 3****.** Scanning Electron Microscopy (SEMI) evaluation of BAMS and Acellular Control sheets, both non-dehydrated and dehydrated. **(A)** BAMS non-dehydrated; **(B)** Acellular Control non-dehydrated; **(C)** BAMS dehydrated; **(D)** Acellular Control dehydrated. n =3. Scale bar: 10 µm, 20 µm and 50 µm. BAMS: Bioengineered Artificial Mesenchymal Sheets.

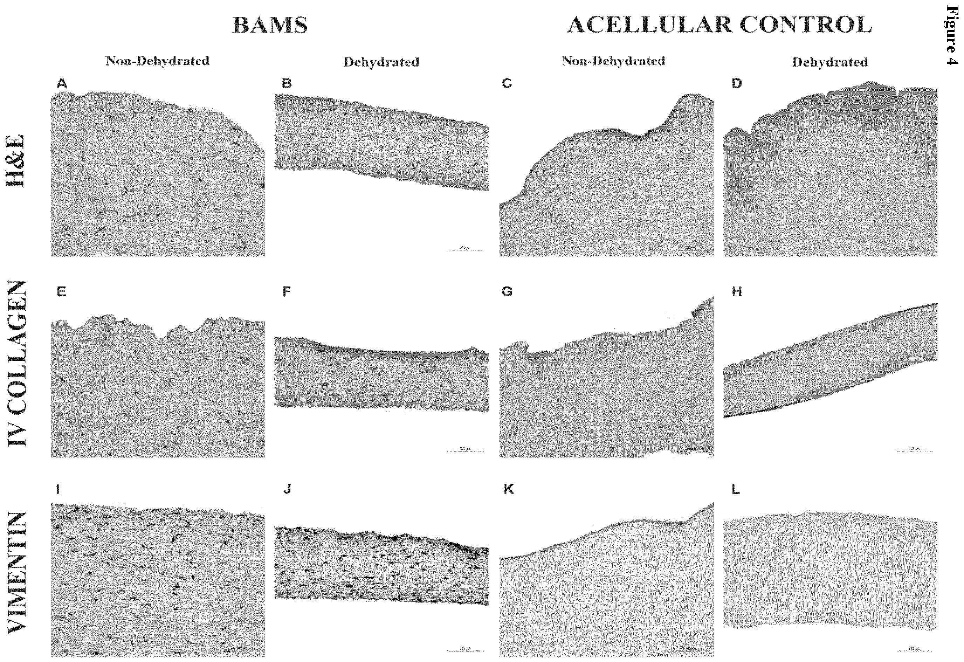

**Figure 4****.** Histological and immunohistochemical analysis of the Acellular Control and BAMS, both non-dehydrated and dehydrated. **(A-D)** Staining with hematoxylin-eosin (H&E). **(E-H)** Immunostaining with collagen IV. **(I-L)** Immunostaining with vimentin. **(A, E, I)** Non-dehydrated BAMS. **(B, F, J)** Dehydrated BAMS. **(C, G, K)** Non-dehydrated Acellular Control. **(D, H, L)** Dehydrated Acellular Control. n =3. Scale bar: 200 µm. BAMS: Bioengineered Artificial Mesenchymal Sheets.

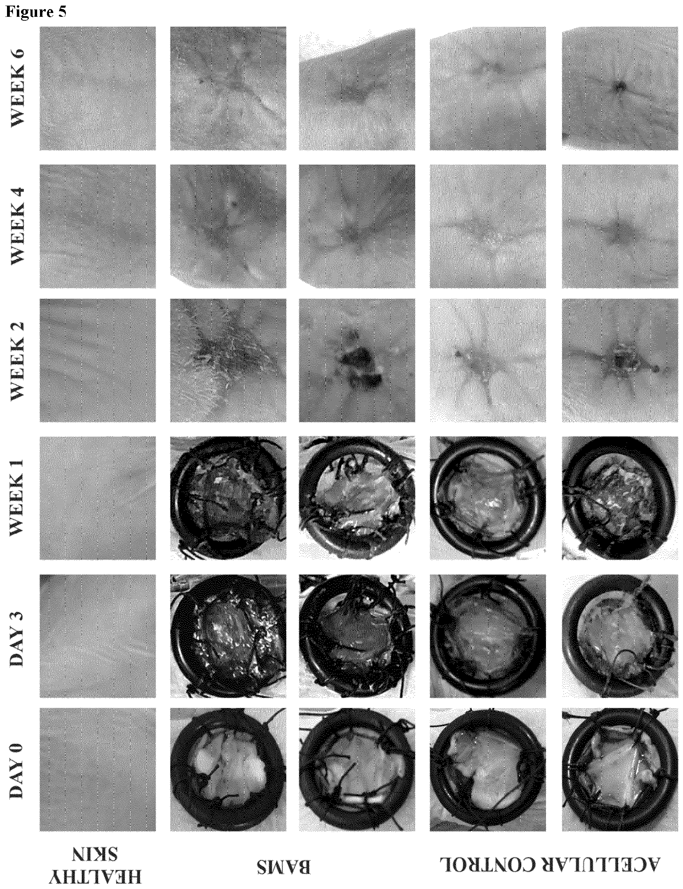

**Figure 5****.** Representative macroscopic images of the *in vivo* wound healing process over time in both groups of mice: Acellular Control and BAMS. In the first row is the healthy skin, in the second and third rows is the BAMS group, and while in the fourth and fifth rows is the Acellular Control group. In each column, the time periods evaluated are represented (day 0, day 3, week 1, week 2, week 4 and week 6). BAMS: Bioengineered Artificial Mesenchymal Sheets.

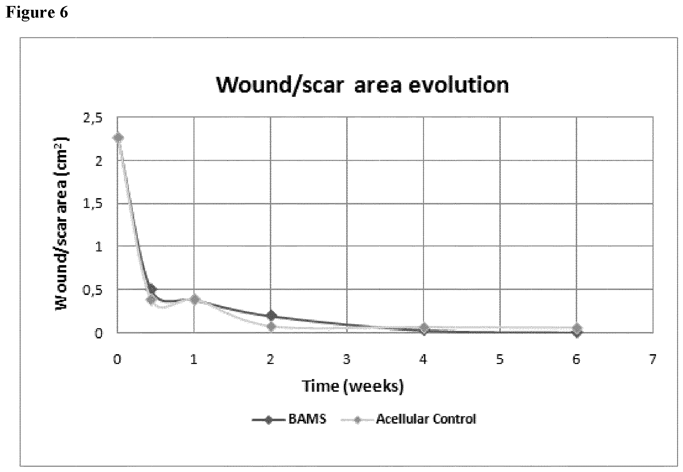

**Figure 6****.** Quantitative assessment of wound/scar area over time for each treatment group: BAMS (blue) and Acellular Control (pink). BAMS: Bioengineered Artificial Mesenchymal Sheets.

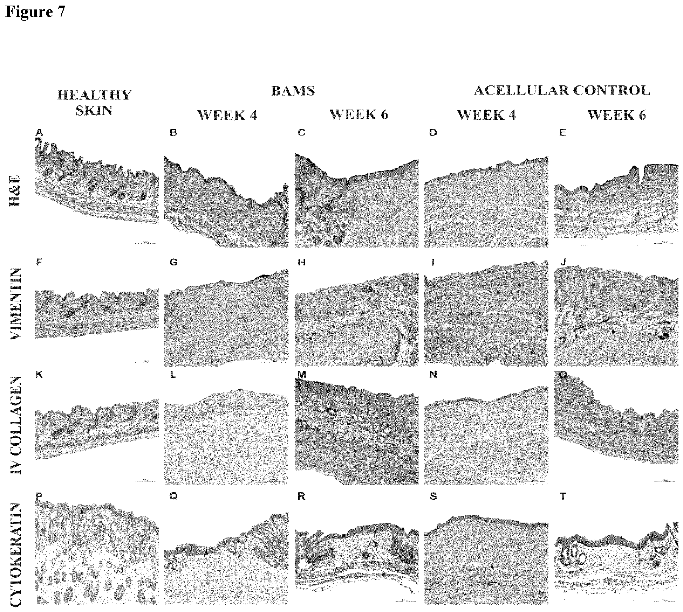

**Figure 7****.** Histological and immunohistochemical staining of mouse skin biopsies 4 and 6 weeks after implantation of the different sheets studied. **(A-E)** Staining with hematoxylin-eosin (H&E). **(F-J)** Immunostaining with vimentin. **(K-O)** Immunostaining with collagen IV. **(P-T)** Immunostaining with cytokeratin. **(A, F, K, P)** Healthy Skin. **(B, G, L, Q)** BAMS group after 4 weeks **(C, H, M, R)** BAMS group after 6 weeks **(D, I, N, S)** Acellular Control group after 4 weeks **(E, J, O, T)** Acellular Control group after 6 weeks. Scale bar: 200 µm. BAMS: Bioengineered Artificial Mesenchymal Sheets.

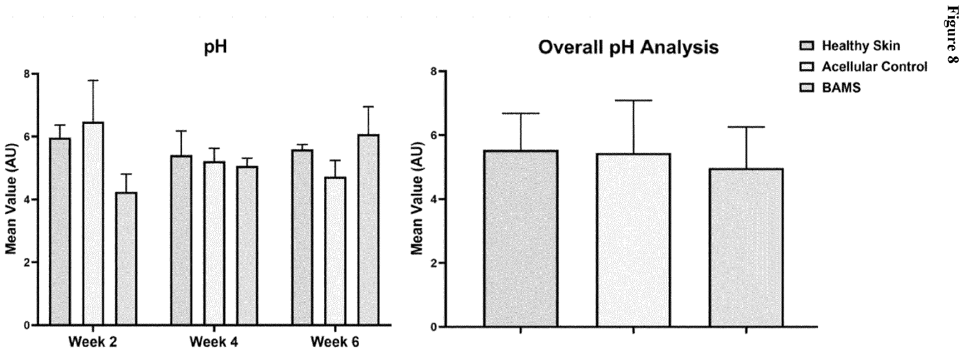

**Figure 8****.** pH analysis using the pHmeter^{®} probe, both globally and per week, representing the mean value with its standard error of the mean. Results per week were calculated as the mean value of all mouse measurements at each time point of the study: Week 2 (n=6), Week 4 (n=6) and Week 6 (n=3). The absence of asterisks means that there are no significant differences. BAMS: Bioengineered Artificial Mesenchymal Sheets.

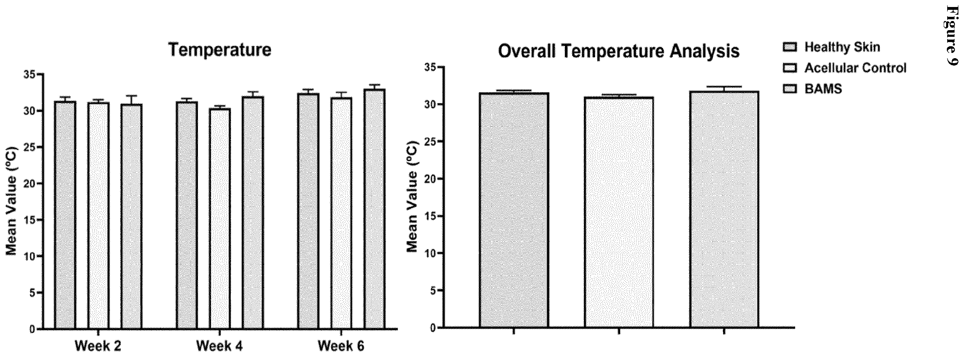

**Figure 9****.** Temperature analysis using the Thermometer^{®} probe, both globally and by week, representing the mean value with its standard error of the mean. Results per week were calculated as the mean value of all mouse measurements at each time point of the study: Week 2 (n=6), Week 4 (n=6) and Week 6 (n=3). The absence of asterisks means that there are no significant differences. BAMS: Bioengineered Artificial Mesenchymal Sheets.

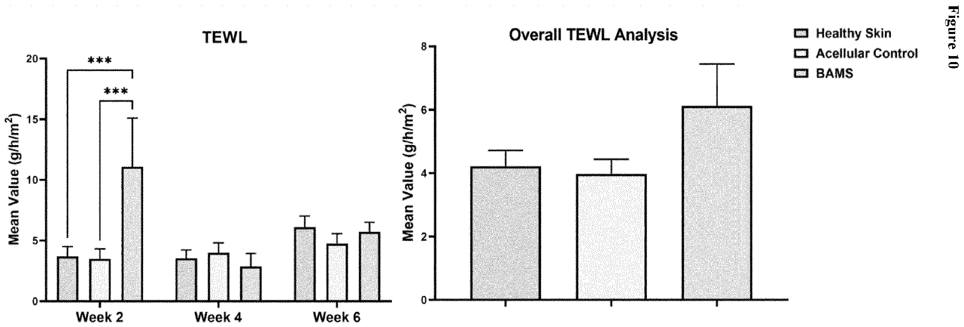

**Figure 10****.** Analysis of transepidermal water loss (TEWL) using the Tewameter^{®} probe, both globally and by week, representing the mean value with its standard error of the mean. Results per week were calculated as the mean value of all mouse measurements at each time point of the study: Week 2 (n=6), Week 4 (n=6) and Week 6 (n=3). Statistical significance: ***p<0.001.
   Absence of asterisks means no significant difference. BAMS: Bioengineered Artificial Mesenchymal Sheets.

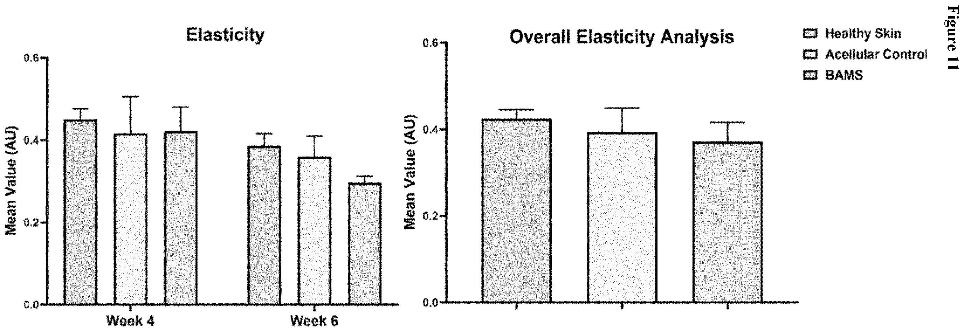

**Figure 11****.** Elasticity analysis using the Cutometer^{®} probe, both globally and per week, representing the mean value with its standard error of the mean. Results per week were calculated as the mean value of all mouse measurements at each time point of the study: Week 4 (n=6) and Week 6 (n=3). The absence of asterisks means that there are no significant differences. BAMS: Bioengineered Artificial Mesenchymal Sheets.

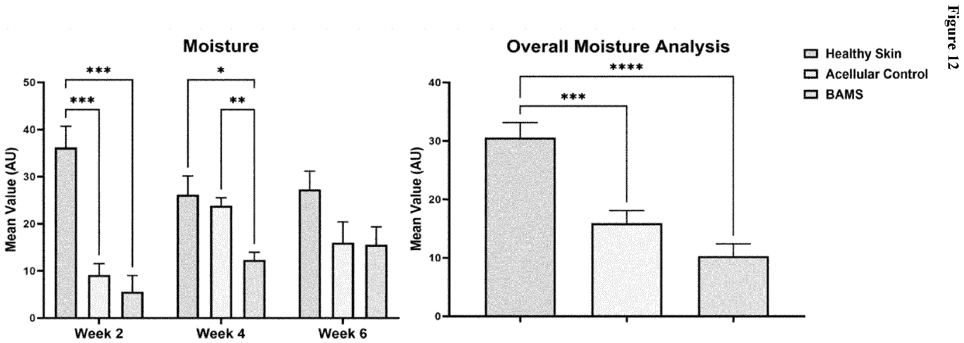

**Figure 12****.** Moisture analysis using the Corneometer^{®} probe, both globally and by week, representing the mean value with its standard error of the mean. Results per week were calculated as the mean value of all mouse measurements at each time point of the study: Week 2 (n=6), Week 4 (n=6) and Week 6 (n=3). Statistical significance: *p<0.05; **p<0.01; ***p<0.001; ****p<0.0001. Absence of asterisks means no significant difference. BAMS:
   Bioengineered Artificial Mesenchymal Sheets.

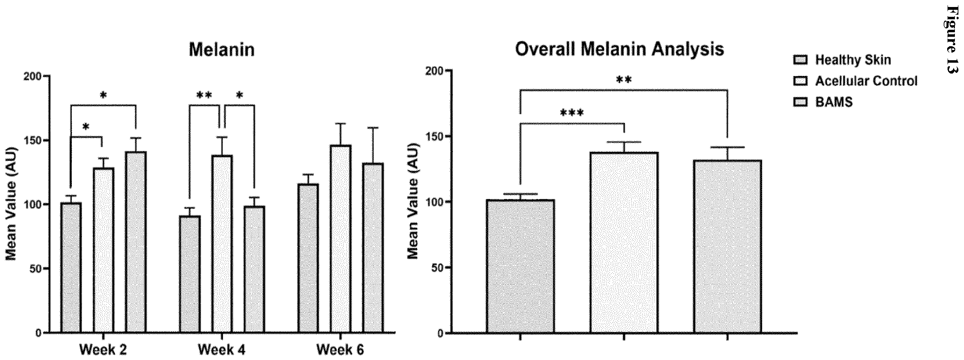

**Figure 13****.** Analysis of pigmentation (melanin) using the Mexameter^{®} probe, both globally and by week, representing the mean value with its standard error of the mean. Results per week were calculated as the mean value of all mouse measurements at each time point of the study: Week 2 (n=6), Week 4 (n=6) and Week 6 (n=3). Statistical significance: *p<0.05; **p<0.01; ***p<0.001. Absence of asterisks means no significant difference. BAMS: Bioengineered Artificial Mesenchymal Sheets.

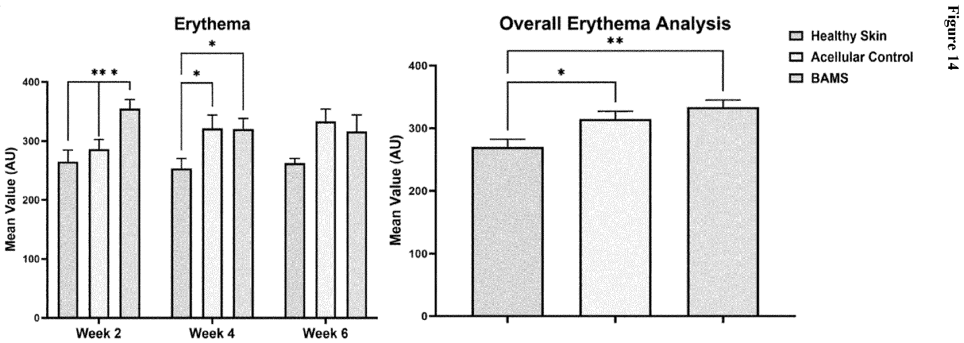

**Figure 14****.** Erythema analysis using the Mexameter^{®} probe, both globally and by week, representing the mean value with its standard error of the mean. Results per week were calculated as the mean value of all mouse measurements at each time point of the study: Week 2 (n=6), Week 4 (n=6) and Week 6 (n=3). Statistical significance: *p<0.05; **p<0.01.
   Absence of asterisks means no significant difference. BAMS: Bioengineered Artificial Mesenchymal Sheets.

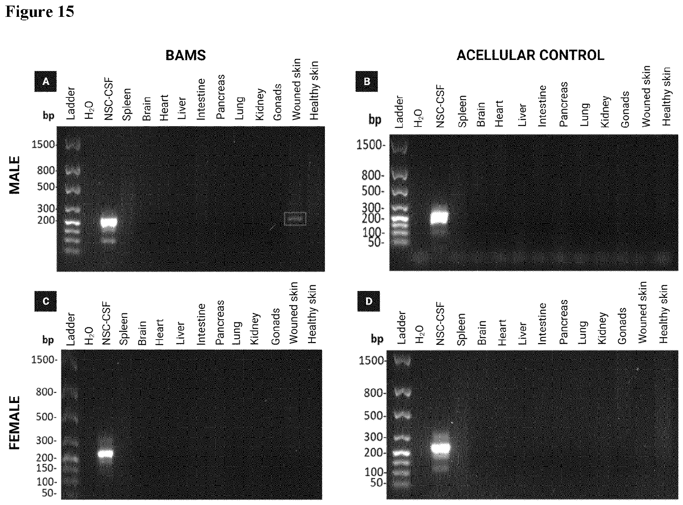

**Figure 15****.** PCR determination of the biodistribution of mesenchymal stem cells in the different murine organs after 6 weeks of evolution. The band observed in column 3 corresponds to the human mesenchymal cells used as positive control (NSC-CSF). **(A)** BAMS Male Mouse; **(B)** Acellular Control Male Mouse; **(C)** BAMS Female Mouse and **(D)** Acellular Control Female Mouse. BAMS: Bioengineered Artificial Mesenchymal Sheets.

### Detailed description of the invention

The present invention is illustrated by means of the Examples set below without the intention of limiting its scope of protection.

### Example 1. Product preparation

For the preparation of the sheet, a mixture containing the active substance of expanded allogeneic adult allogeneic adipose tissue Mesenchymal Trunk Mesenchymal Cells and its excipients will be generated. A first tube is prepared with the following components: the cell suspension volume, human plasma, complete culture medium and tranexamic acid. Once prepared, it is stored in the incubator until use. Subsequently, a second tube is prepared with calcium chloride, water for injectables and hyaluronic acid, to which the previous mixture is added, producing the final mixture that will form the sheet. This mixture is quickly added to the culture plates so that it gels (MP-005- FOR05 "Manufacture of ulcer sheets"). Two sheets with cells are produced: one for the investigational drug (21cm²), and one for quality control (1.4 cm²).

### Example 2. Allogenic adipose-derived adult mesenchymal stem cells expanded on fibrin-hyaluronic biological matrix.

The allogenic adipose-derived adult mesenchymal stem cells (ADSC) expanded on fibrin-hyaluronic biological matrix, particularly on Bioengineered Artificial Mesenchymal Sheet (BAMS).

BAMS is a biomaterial matrix (comprising fibrin and hyaluronic acid) containing embedded expanded allogeneic adult mesenchymal stem cells from adipose tissue (ADSCs) at a concentration of about 360,000 cells/cm². The BAMS has a surface area of 21 cm² and contains a total of 7.5 × 10⁶ ADSCs.

BAMS particularly comprises:
- Active Substance: Allogenic adipose-derived adult mesenchymal stem cells expanded.
- Excipients: Hyaluronic acid, human plasma (fibrin), culture medium, tranexamic acid, CaCl₂ and water. Hyaluronic acid is a polysaccharide of the β-linked glycosaminoglycan type, which is notable for its structural function, thanks to which the sheet acquires the consistency of a gel. The gel obtained forms a three-dimensional network of polymeric fibres. Tranexamic acid is an antifibrinolytic that blocks the lysine present in the active site of plasmin, allowing the three-dimensional scaffolds generated to last longer without significantly altering their properties. Calcium chloride is a coagulation inducer promoting the formation of the three-dimensional networks.

**Table 3**

| **EXCIPIENTS** | **VOLUME (BAMS V_{TOTAL} =10 mL)** |
|---|---|
| Hyaluronic acid (final concentration 0.08%) | 0.5 mL |
| Human plasma | 8.3 mL |
| Culture medium | 0.624 mL |
| Tranexamic acid | 0.202 mL |
| 2% CaCl₂ | 0.240 mL |
| Water for injection | 0.134 mL |

| | |
|---|---|
| *BAMS composition* | |

### Example 3. Batch formulation

The formulation per batch shall contain 1 BAMS, 1 sheet of allogeneic adult mesenchymal stem cells from adipose tissue expanded in a biological matrix of fibrin-hyaluronic acid, containing 7.5 × 10⁶ embedded cells, at a concentration of 360,000 cells/cm², with a surface area of 21 cm².

### Example 4. Pharmaceutical development

The concentration of 7.5 ×10⁶ cells/sheet was chosen on the basis of preliminary studies indicating that no cell aggregates are formed and that it is an optimal range for the stability of the sheet.

During the fine-tuning of the process of obtaining the sheet, several tests were carried out to determine the most suitable cell concentration.

**Table 4** shows the results obtained in the first of these tests. For the production of a sheet with a volume of 10 ml, 0.624 ml of cell solution was used. Different concentrations of the cell solution, 12, 20 and 25 ×10⁶ ADSCs/ml, were tested to see if the total number of cells embedded in a sheet, 7.5×10⁶, 12.5×10⁶ and 15.6×10⁶ cells respectively, has an impact on the gelation process, integrity and stability of the sheet. The tests were carried out with CORNING plates (reference 430166).

The sheets with the highest number of cells, 12.5×10⁶ and 15.6×10⁶ total cells, did not gel well, macroscopically did not show adequate stability and degraded. So, plates with up to 7.5 × 10⁶ total cells are preferred.

**Table 4**

| **Batch** | **Cell viability** | **Cell Concentration (ADSCs/ml)** | **Volume cell suspension** | **Amount cell/sheet** | **Optimal gelification** |
|---|---|---|---|---|---|
| 1 | 93.3% | 12×10⁶ | 0.624ml | 7.5 × 10⁶ | Yes |
| 2 | 93.3% | 20×10⁶ | 0.624ml | 12.5 × 10⁶ | No |
| 3 | 93.3% | 25×10⁶ | 0.624ml | 15.6 × 10⁶ | No |

| | | | | | |
|---|---|---|---|---|---|
| *Evaluation of cell concentration of the sheets in the gelation process.* | | | | | |

The amount of cells present in a sheet is directly related to its stability, since the greater the number of cells, the faster the degradation process of its. Once the cell concentration at which the sheet has been determined, it is necessary to test the viability cells over time. Three cell concentrations were tested, and viability tests were performed by Live&Dead at 24h and 48h (**Table 5**). After verifying that the 3 concentrations gelled correctly and maintained optimal viability at 48h, we chose the cell concentration with the highest dose.

**Table 5**

| **Batch** | **Cell viability** | **Cell Concentration (ADSCs/ml)** | **Volume cell suspension (5ml sheet)** | **Amountcell/s heet** | **Correct gelification** | **BAMS Viability** | |
|---|---|---|---|---|---|---|---|
| | | | | | | **24 h** | **48h** |
| 1 | 98.1% | 12×10⁶ | 0.312 ml | 3.7×10⁶ | Yes | 92.7% | 85% |
| 2 | 98.1% | 9×10⁶ | 0.312 ml | 2.8×10⁶ | Yes | 84.2% | 91.1% |
| 3 | 98.1% | 6×10⁶ | 0.312 ml | 1.8×10⁶ | Yes | 84.2% | 78.9% |

| | | | | | | | |
|---|---|---|---|---|---|---|---|
| *Study of the stability of the sheets.* | | | | | | | |

### Example 5. Control of the investigational medicinal product

### Example 5.1. Product specifications

The final product specifications are detailed in the following **Table 6:**

**Table 6**

| **Specifications Quality attribute** | **Method/ Reference** | **Acceptance requirements** |
|---|---|---|
| Pre-release sterility | Ph. Eur. 2.6.27 | Absence of microorganisms |
| Sterility⁽¹⁾ | Ph. Eur. 2.6.27 | Absence of microorganisms |
| Endotoxins | Ph.Eur. 2.6.14 | <12 EU/ml |
| Mycoplasma | Ph.Eur. 2.6.7 | Absence of mycoplasma |
| Viability | *Live &Dead* staining | ≥ 70% |

(1) The use of the finished product has a stability of 6 hours after packaging. For this reason, it is not possible to wait for the definitive results of the sterility test before releasing the product. An early release of the medicinal product is carried out in order to proceed with the implantation in the patient, while waiting for the definitive results of the sterility test. Once the definitive results are obtained, they are attached to the batch documentation and the definitive release certificate is issued.

### Example 6. Preclinical phase

### Example 6.1. Methodology

The study comprises the evaluation of the BAMS (Bioengineered Artificial Mesenchymal Sheets) product in order to determine the suitability of the application in mice through different approaches: clinical, histological and skin homeostasis evaluation. This is a non-clinical *in vivo* trial with a design similar to the clinical trial in patients with chronic ulcers.

The BAMS product comprises a hyaluronic acid-fibrin sheet containing mesenchymal cells obtained from adipose tissue samples from donors by surgical excision. Once in the laboratory, the tissue was washed with a solution consisting of Dulbecco's phosphate buffered saline solution (DPBS), gentamicin and vancomycin. After this, in order to isolate the mesenchymal cells, mechanical disaggregation of the tissue was combined with enzymatic digestion. Once isolated, the cells were cultured for expansion at 37°C and 5% CO₂. It should be noted that all human samples were obtained with the informed consent of the patient, as well as in accordance with the ethical standards of the committee responsible for human experimentation (Provincial Ethical Committees of Granada) and with the Helsinki Declaration of 1975, revised in 1983.

For the manufacture of the BAMS product, different reagents (described in **Table 7)** were used to achieve a final volume per well of 5 mL (wells of 9.6 cm² diameter) and a cell concentration of 7 × 10⁵ cells/mL.

**Table 7**

| | **Reagents** | **Volume (ml) (V_{Total}= 5 mL/sheet)** |
|---|---|---|
| **Tube 1** | **Plasma** | **4.150** |
| | **Cell suspension + Culture medium** | **0.312** |
| | **Tranexamic acid** | **0.101** |
| **Tube 2** | **Calcium chloride** | **0.120** |
| | **Water** | **0.067** |
| | **Hyaluronic acid** | **0.250** |

| | | |
|---|---|---|
| *Reagents used for the manufacture of the mesenchymal cell-based sheet, BAMS.* | | |

Tube 1 was poured into tube 2, after which it was rapidly distributed into 6-well culture plates, which were incubated at 37°C and 5% CO₂ for 1 week prior to implantation into the mice. In addition, the same type of sheets without cellular component (here after referred to as acellular control) was used as a control for the study. After that week and prior to implantation, both sheets were subjected to a pressure dehydration process using a 300 g glass disc for 2 minutes. At the time of the operation, each slice was divided in half to cover the size of the wound.

For the *in vivo* evaluation of the product, a total of 12 4-week-old BALB/cAnNRj-Foxn1^{*nu*/*nu*} male and female mice (6 for the BAMS group and 6 for the acellular control group), weighing approximately 20 grams, immunodeficient, athymic, nude and albino, were used (**Figure 1**). The study was approved by the Provincial Ethics Committee of Granada and was carried out in compliance with good laboratory practice standards, as well as the ethical principles, regulations and guidelines applicable to animal experimentation.

First, surgery was performed to remove an area of skin using a 17 mm diameter packing ring to standardise the size of the wound. This was performed on the upper dorsum of the animal longitudinal to the spine of the mouse using a scalpel and scissors. In addition, the packing ring was sewn to the edges of the wound to prevent the wound from closing by the natural process of muscle contraction in mice. In this way, we could be sure that the healing process was mainly due to the study sheets. For this purpose, the animals were anesthetized by intraperitoneal injection of ketamine 50 mg/mL (2.5 mg/mL), xylazine 20 mg/mL (0.2 mg/mL) and acepromazine 5 mg/mL (0.05 mg/mL). After this, the corresponding sheet (BAMS or Acellular Control) were implanted and, in order to protect the wound, they were covered with antibiotic ointment (Blastoestimulin), Mepitel and bandage which was removed after 3 or 4 days. In addition, an anti-inflammatory drug (meloxican 5 mg/mL, 0.02 mL/mouse) was injected subcutaneously as postoperative treatment.

To study the wound evolution, an exhaustive clinical follow-up for 6 weeks was performed, observing the macroscopic integration of engraftment. Moreover, an adaptation of the "Patient and Observer Scar Assessment" (POSAS) scale was used. For this purpose, different parameters (vascularity, pigmentation, thickness, relief, flexibility and surface area) were evaluated at different times (day of surgery [0 day], 3 days, 1 week, 2 weeks, 4 weeks and 6 weeks). The above parameters have to be given a value for the area of sheet implantation between 1-10 taking the adjacent healthy skin as a reference, with 1=normal skin and 10=worst imaginable scar.

Every 2 weeks (week 2, week 4 and week 6) different skin homeostasis parameters were evaluated, both in the area where the graft had been implanted (wound) and in the surrounding healthy skin of the mouse using the probe system Microcaya (Microcaya S.L., Bilbao, Spain): Thermometer^{®} (measured infrared temperature in °C), pHmeter^{®} (determined pH of skin using a glass rod with all elements required to measure it), Tewameter^{®} (measured transepidermal water loss (TEWL) or evaporation of water in g/h/m² based on the principle of diffusion in an open chamber), Cutometer^{®} (evaluated skin elasticity by suction in arbitrary units (AU)), Corneometer^{®} (determined moisturization through the capacitance of a dielectric medium in AU), and Mexameter^{®} (measured erythema giving indirect information on skin vascularization [hemoglobin levels] as well as pigmentation [melanin level]). Prior to the measurements, the mice were anesthetized to avoid unnecessary stress by inhalation of 2% Sevofluorane.

Finally, half of the mice in each group were sacrificed after 6 weeks by cervical dislocation (n=3; 2 females and 1 male). From each sacrificed animal, different organs were isolated to study the biodistribution of mesenchymal cells (spleen, brain, heart, liver, intestine, pancreas, lungs, kidneys, and gonads [male and female]). For this purpose, the presence of human cells was analysed by PCR detection of human ALU sequences in samples from the above-mentioned organs, tissue obtained from wound skin, and healthy skin. The following steps were followed: 1) Homogenisation and isolation of DNA from each sample; 2) Determination of the presence of ALU sequences by PCR according to the methodology described by Garcia-Delgado AB, 2019, and 3) Estimation of the number of cells per milligram of tissue from a standard curve made from human cell DNA. A mesenchymal cell suspension was used as a positive control (NSC-CSF).

In addition, biopsies were collected from the area where the sheet had been implanted that also included surrounding healthy skin for clinical evaluation. The biopsies were fixed in 4% formaldehyde, dehydrated, embedded in paraffin and cut into 4 µm sections at the Anatomical Pathology Department of the Hospital Universitario Virgen de las Nieves in Granada. The sections were stained with hematoxylin and eosin (H&E) and immunohistochemistry (vimentin, cytokeratin and collagen IV) to assess histological structure.

Simultaneously, *in vitro* characterisation of the sheet at histological and microstructural level was performed. Histological staining (hematoxylin and eosin staining) and immunohistochemistry (vimentin and collagen type IV) were used to determine the distribution of mesenchymal cells. Moreover, cell arrangement was determined by confocal microscopy and cell viability was assessed with the LIVE/DEAD viability/cytotoxicity kit following the manufacturer's recommendations. Finally, the internal structure was visualised by scanning electron microscopy.

GraphPad software was used for statistical analysis. All data were expressed as mean ± standard error of the mean (SEM). Statistical significance was calculated by one-way ANOVA after which a Tukey analysis for multiple comparisons was performed.

### Example 6.2. Results

The *in vitro* characterisation of the sheets used was carried out with different techniques.

**Figure 2** shows representative images of the BAMS sheet obtained in the LIVE/DEAD viability assay both non-dehydrated (cell viability = 98.1 %) and dehydrated (cell viability = 96.2 %).

The cell distribution along the non-dehydrated and dehydrated BAMS product was assessed by scanning electron microscopy (SEMI). In these images, more elongated cells of mesenchymal morphology were found. Acellular control is shown the cell-free structure (**Figure 3**).

H&E staining was used to define the cells structural distribution in the sheet before and after pressure dehydration (Figure 4-A, B). Collagen IV immunostaining shows a normal distribution of mesenchymal cells throughout the sheet. It is the main collagen component of the basement membrane involved in important physiological and pathological functions (**Figure 4**-E, F). Similarly, vimentin is a filament protein normally present in eukaryotic mesenchymal cells making them stiff and tough. Therefore, immunostaining of the sheets using an antibody directed to this protein showed that the cells maintain their phenotype within the BAMS product both before and after dehydration (Figure 4-I, J). Acellular control was used as a negative control (Figure 4-C, D, G, H, K, L).

The *in vivo* assessment showed a figure that includes the clinical assessment of wound regeneration for both groups (**Figure 5**), as well as the values assigned to the POSAS scale (**Table 8**).

**Figure 5** demonstrated that physical separation was maintained for one week, with adequate biointegration observed after 2 weeks in both sheets evaluated (Acellular Control and BAMS). At week 4, it appears that the wound healing and skin regeneration process is slower in the case of the Acellular Control, with even a more aesthetically acceptable scar being observed for the BAMS group.

Visual clinical evaluation was correlated with quantitative analysis of wound/scar area (**Figure 6**), where significant differences were observed between the day of the surgery (day 0) and the rest of the weeks in both groups (p-value < 0.0001).At week 2, it appears that the Acellular Control has a smaller wound area, and thus better wound healing capacity compared to BAMS. However, at the end of the study (week 6) only in BAMS group the wound area was almost disappeared.

Assessment of scars was made using an adaptation of the POSAS scale (**Table 8**). Total score revealed that BAMS (10) presented better results than the Acellular Control (13).

**Table 8**

| | **Vascularity^{a}** | **Pigmentation^{a}** | **Thickness^{a}** | **Relief^{a}** | **Pliability^{a}** | **Surface area^{a}** | **Total score^{b}** |
|---|---|---|---|---|---|---|---|
| ***Acellular Control*** | 3 | 2 | 2 | 2 | 2 | 2 | 13 |
| ***BAMS*** | 2 | 3 | 2 | 1 | 1 | 1 | 10 |

| | | | | | | | |
|---|---|---|---|---|---|---|---|
| *Results of the POSAS scale after 6 weeks of evaluation. BAMS: Bioengineered Artificial Mesenchymal Sheets.* *^{a}All items are scored on a scale ranging from 1 ('like normal skin') to 10 (worst imaginable scar').* *^{b}The lowest score, 6, reflects normal skin, whereas the highest score, 60, reflects the worst imaginable scar.* | | | | | | | |

H&E staining (**Figure 7**) of engraftments in both groups of mice, (Acellular Control and BAMS revealed a correct integration and regeneration of the epidermis and dermis after 4 weeks. However, BAMS group (Figure 7- B, G, L, Q) presented a more complex structure, composed of more cells in dermal matrix than the Acellular Control group (Figure 7-D, I, N, S). As expected, after 6 weeks of study, the structure was more defined in both sheets tested as the wound regeneration was greater, with virtually no area of injury.

Homeostasis parameters were studied at different evaluation periods using the Microcaya probe system. All groups were compared with healthy skin of mice.

pH values (**Figure 8**) were like to healthy skin in all groups and weeks, without notable differences at 6 weeks. In the case of temperature (**Figure 9**), values were homogeneous and similar to healthy skin results in all groups, and no significant differences were observed through the study.

Transepidermal water loss (TEWL) is an important parameter in clinical field. It is related to the level of hydration of the skin, which is one of the main functions of the skin that is affected when there is damage or injury, hence the importance of being able to achieve rapid recovery. The results showed higher values at week 2 for the BAMS group which is puzzling. However, the barrier was recovered after 6 weeks of evaluation since no significant differences were observed between groups compared to healthy skin. Furthermore, in the overall analysis, no significant differences were observed between the two sheets or with healthy skin. This could indicate that the high loss observed in the BAMS group at week 2 could be due to external factors and may not be conclusive (**Figure 10**).

Elasticity values **(****Figure 11****)** were homogeneous and similar to healthy skin results in both groups, and no significant differences were observed through the study. Measurements were not taken at week 2 as the probe performs a slight suction of the skin and, given that at that time the wound of some mice had not completely healed, the measurement could give erroneous values and the animal would be made to suffer more.

In the case of moisture **(****Figure 12****),** the results revealed significant differences between the two sheets evaluated (Acellular Control and BAMS) at both weeks 2 and 4 compared to healthy skin. However, this difference disappears at week 6, stabilising the values of moisture. Furthermore, overall moisture analysis graph showed no significant differences between the two groups.

The last parameters evaluated within homeostasis were erythema and melanin (pigmentation). In the case of melanin, at week 2 significantly higher values were observed in both groups compared to healthy skin. This is indicative that the skin is healing. These significant differences were absent at week 6, and the values stabilise. In the overall analysis, significant differences were observed between BAMS and Acellular Control compared to healthy skin **(****Figure 13****).**

Erythema analysis, similar to melanin, showed at week 2 significantly higher values in both sheets compared to healthy skin. This correlates with phase 2 of the wound healing process, known as inflammation, where reddening of the skin and increased vascularization occurs. Again, at week 6, these significant differences with healthy skin disappeared for both groups, obtaining more homogeneous values. In the overall analysis, significant differences were observed between BAMS and the Acellular Control in comparison with healthy skin (**Figure 14**).

The biodistribution study confirmed that there had been no cell migration to any of the organs evaluated, neither in the Acellular Control group (as expected) nor in the BAMS group. However, in the BAMS group, the presence of cellular DNA was observed in the skin corresponding to the wound area (**Figure 15**). This shows the integration of the study sheet in the wound area. The absence of human mesenchymal cells in both the female and male gonads is noteworthy, ensuring no transmission of genetic material to the next generation.

Based on the various results described throughout this patent application, it appears that the use of the BAMS product improves the efficiency of the wound healing and skin regeneration process in an immunocompromised subject.

## Claims

1. Composition comprising mesenchymal stem cells as active substance expanded within a cell matrix, **characterized in that** the cell matrix comprises: Hyaluronic acid and fibrin.

2. Composition, according to claim 1, wherein the cell matrix comprises: Hyaluronic acid, human plasma, tranexamic acid and CaCl₂.

3. Composition, according to any of the previous claims, wherein the cell matrix comprises culture medium and water.

4. Composition, according to any of the previous claims, wherein the matrix comprises: between 1 and 10 % v/v of hyaluronic acid and between 78 and 88 % v/v of human plasma.

5. Composition, according to any of the previous claims, wherein the matrix comprises: between 1 and 10 % v/v of hyaluronic acid, between 78 and 88 % v/v of human plasma, 2.02 % v/v of culture medium, 2.4 % v/v of tranexamic acid, 1.34 % v/v of CaCl₂ and 6.24 % v/v of water.

6. Composition, according to any of the previous claims, wherein the matrix comprises: 5 % v/v of hyaluronic acid, 83 % v/v of human plasma, 2.02 % v/v of culture medium, 2.4 % v/v of tranexamic acid, 1.34 % v/v of CaCl₂ and 6.24 % v/v of water.

7. Composition, according to any of the previous claims, wherein the matrix is in the form of a gel.

8. Composition, according to any of the previous claims, **characterized in that** the mesenchymal stem cells are obtained from adipose tissue, preferably from subcutaneous adipose tissue.

9. Composition, according to any of the previous claims, **characterized in that** the concentration of the mesenchymal stem cells expanded within the cell matrix is between 330,000 cells/cm² and 400,000 cells/cm².

10. Composition, according to any of the previous claims, **characterized in that** the concentration of the mesenchymal stem cells expanded within the matrix is 360,000 cells/cm².

11. Composition, according to any of the previous claims, **characterized in that** it is in the form of a sheet having between 2 and 144 cm² and a total amount of cells between 330,000 cells/cm² and 400,000 cells/cm².

12. Composition, according to any of the previous claims, **characterized in that** it is in the form of a sheet having 21 cm² and a total amount of 7.5×10⁶ cells.

13. Composition, according to any of the previous claims, for use in tissue repair or tissue regeneration.

14. Composition for use, according to claim 13, in the treatment of venous ulcers.

15. Composition for use, according to claims 13 or 14, **characterized in that** it is administered topically.
